# EUROPEAN PATENT APPLICATION

(11) **EP 4 635 490 A1**
(43) Date of publication of application: **22.10.2025**
(21) Application number: 24382410.9
(22) Date of filing: 18.04.2024
(51) Int. Cl.: A61K 31/198, A61K 31/201, A61P 25/16, A61P 25/28

(54) **PM20D1-DERIVED N-OLEOYL-L-LEUCINE FOR THE TREATMENT AND PREVENTION OF NEURODEGENERATIVE DISEASES**

(71) Applicant: Consejo Superior De Investigaciones Científicas, 28006 Madrid (ES)
(72) Inventor: Sanchez Mut, Jose Vicente, Alicante (ES); Gräff, Johannes, Lausanne (CH)
(74) Representative: Pons IP

(57) **Abstract**

The invention relates to PM20D1-derived N-oleoyl-L-Leucine (C18:1-Leu) for the treatment and/or prevention of neurodegenerative diseases, particularly for the treatment and/or prevention of Parkinson disease, multiple sclerosis disease, amyotrophic lateral sclerosis disease and Alzheimer's disease.

## Description

The invention relates to PM20D1-derived N-oleoyl-L-Leucine (C18:1-Leu) for the treatment and/or prevention of neurodegenerative diseases, particularly particularly for the treatment and/or prevention of Parkinson disease, multiple sclerosis disease, amyotrophic lateral sclerosis disease and Alzheimer's disease.

### BACKGROUND ART

Alzheimer's disease (AD) is an age-related neurodegenerative disorder characterized by a progressive decline in cognitive abilities and the presence of two types of proteinaceous aggregates, amyloid plaques and neurofibrillary (Scheltens, et al., Alzheimer's disease. Lancet 397, 1577-1590 (2021). AD is believed to be the consequence of a complex interaction between genetic and non-genetic risk factors, that are usually investigated using genome- (GWAS) and epigenome- (EWAS) wide association studies, respectively. Combining both types of studies, we and others have recently identified a new AD risk region centered on the *Peptidase M20 Domain Containing 1* (PM20D1) (see Sanchez-Mut, J. V. et al. PM20D1 is a quantitative trait locus associated with Alzheimer's disease. Nat Med 24, (2018); Wang, Q. et al. Longitudinal data in peripheral blood confirm that PM20D1 is a quantitative trait locus (QTL) for Alzheimer's disease and implicate its dynamic role in disease progression. Clin Epigenetics 12, 189 (2020); Smith, R. G. et al. A meta-analysis of epigenome-wide association studies in Alzheimer's disease highlights novel differentially methylated loci across cortex. Nat Commun 12, 3517 (2021); Li QS et al. Association of peripheral blood DNA methylation level with Alzheimer's disease progression. Clin Epigenetics 13, 191 (2021); Coto-Vilchez, C. et al. Genome-wide DNA methylation profiling in nonagenarians suggests an effect of PM20D1 in late onset Alzheimer's disease. CNS Spectr 1-9 (2021); and Pérez, R. F. et al. Blood DNA methylation patterns in older adults with evolving dementia. J Gerontol A Biol Sci Med Sci (2022)). Additional evidence of PM20D1 locus association with Parkinson's disease (PD) and Multiple Sclerosis (MS) has also been reported (Satake et al. Genome-wide association study identifies common variants at four loci as genetic risk factors for Parkinson's disease. Nat Genet. 41(12):1303-7 (2009). Maltby et al. Differential methylation at MHC in CD4+T cells is associated with multiple sclerosis independently of HLA-DRB1. Clin Epigenetics. 9:71 (2017)). Human low expression carriers of PM20D1 have a higher risk to develop AD, while PM20D1 overexpression in mouse models improves glucose metabolism and reduces amyloid burden and cognitive deficits. PM20D1 is a secreted enzyme that regulates the conjugation of fatty acids with amino acids, generating a series of compounds named N-acyl amino acids (NAAAs). NAAAs are present in multiple tissues, including brain (see Raboune, S. et al. Novel endogenous N-acyl amides activate TRPV1-4 receptors, BV-2 microglia, and are regulated in brain in an acute model of inflammation. Front Cell Neurosci 8, 195 (2014); and Leishman, E., Mackie, K., Luquet, S. & Bradshaw, H. B. Lipidomics profile of a NAPE-PLD KO mouse provides evidence of a broader role of this enzyme in lipid metabolism in the brain. Biochim Biophys Acta 1861, 491-500 (2016)) and their levels are directly correlated with the expression of PM20D1 in humans and mice (Yang, R. et al. PM20D1 is a circulating biomarker closely associated with obesity, insulin resistance and metabolic syndrome. Eur J Endocrinol 186, 151-161 (2021)). They are believed to act as protective molecules regulating inflammation, ischemia and traumatic brain injury (see Cohen-Yeshurun, A. et al. N-arachidonoyl-L-serine (AraS) possesses proneurogenic properties in vitro and in vivo after traumatic brain injury. J Cereb Blood Flow Metab 33, 1242-1250 (2013); Cheng, L. et al. N-Linoleyltyrosine Protects against Transient Cerebral Ischemia in Gerbil via CB2 Receptor Involvement in PI3K/Akt Signaling Pathway. Biol Pharm Bull 42, 1867-1876 (2019); Burstein, S. H. N-Acyl Amino Acids (Elmiric Acids): Endogenous Signaling Molecules with Therapeutic Potential. Mol Pharmacol 93, 228-238 (2018); Battista, N., Bari, M. & Bisogno, T. N-Acyl Amino Acids: Metabolism, Molecular Targets, and Role in Biological Processes. Biomolecules 9, (2019); Arul Prakash, S. & Kamlekar, R. K. Function and therapeutic potential of N-acyl amino acids. Chem Phys Lipids 239, 105114 (2021); and Bhandari, S., Bisht, K. S. & Merkler, D. J. The Biosynthesis and Metabolism of the N-Acylated Aromatic Amino Acids: N-Acylphenylalanine, N-Acyltyrosine, N-Acyltryptophan, and N-Acylhistidine. Front Mol Biosci 8, (2022)).

Considering all the above, it would be desirable to have new drugs which would be useful for the treatment and/or prevention of neurodegenerative disorders as disease modifying agents, and particularly for the treatment and/or prevention of Parkinson disease, multiple sclerosis disease, amyotrophic lateral sclerosis disease and Alzheimer's disease.

### SUMMARY OF THE INVENTION

The present invention discloses the effects of PM20D1-derived N-oleoyl-L-Leucine (C18:1 -Leu) on the onset and progression of neurodegenerative diseases, particularly Alzheimer's disease (AD), through combining a suite of experimental models, behavioral phenotyping as well as biochemical and molecular techniques. It has been found that the exogenous administration of PM20D1-derived N-oleoyl-L-Leucine (C18:1-Leu) in neuronal cultures from AD mice reduces the production of amyloid-beta (Aβ). PM20D1-derived N-oleoyl-L-Leucine (C18:1 -Leu) treatment in a *C*. *elegans* model of AD also alleviates motility deficits and amyloid aggregation and its supplementation in AD mice ameliorates cognitive deficits and amyloid burden. In line with this, PM20D1-derived N-oleoyl-L-Leucine (C18:1-Leu) treatment in AD mice increases microglia association with amyloid plaques, reducing their number, size and toxicity, and neuronal fitness. These changes are functionally associated with an increase in Aβ chemotaxis and clearance in primary microglia and enhanced neuronal cell viability under reactive oxygen species (ROS) and unfolded-protein stress. Supporting this, it has been found that PM20D1 expression in human samples is positively and negatively correlated with specific transcriptional signatures associated with cognitive reserve and neurofibrillary tangles (NFT), respectively. Taken together, these results provide unprecedented molecular detail on the functional relevance of a recently discovered AD risk factor, and supports the use of PM20D1-derived N-oleoyl-L-Leucine (C18:1-Leu) as disease modifying agents for the treatment of AD.

Accordingly, a first aspect of the present invention relates to N-oleoyl-L-Leucine (C18:1-Leu) for use in the treatment and/or prevention of a neurodegenerative disease, preferably wherein the neurodegenerative disease is selected from Parkinson disease, multiple sclerosis disease, amyotrophic lateral sclerosis disease and Alzheimer's disease, and more preferably wherein the neurodegenerative disease is Alzheimer's disease (AD).

Another aspect of the invention relates to the use of N-oleoyl-L-Leucine (C18:1-Leu) for the manufacture of a medicament for the treatment and/or prevention of a neurodegenerative disease, preferably wherein the neurodegenerative disease is selected from Parkinson disease, multiple sclerosis disease, amyotrophic lateral sclerosis disease and Alzheimer's disease, and more preferably wherein the neurodegenerative disease is Alzheimer's disease (AD).

Another aspect of the invention relates to a method of treating and/or preventing a neurodegenerative disease, preferably wherein the neurodegenerative disease is selected from Parkinson disease, multiple sclerosis disease, amyotrophic lateral sclerosis disease and Alzheimer's disease, and more preferably wherein the neurodegenerative disease is Alzheimer's disease (AD), in a subject in need thereof, especially a human being, which comprises administering to said subject an effective amount of N-oleoyl-L-Leucine (C18:1-Leu).

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

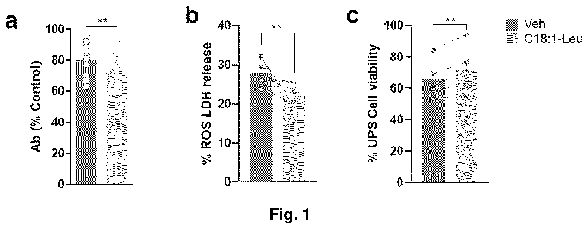

**Fig. 1****.** C18:1-Leu treatment in neuronal cultures. **a,** Enzyme-linked immunosorbent assays (ELISA) of APP/PS1-derived hippocampal neuronal cultures treated during 24h with 200nM C18:1-Leu. n=5 per group, p=0.0093; one-sided Student's t-test. **b,** Lactate dehydrogenase (LDH) release viability assay of APP/PS1-derived hippocampal neuronal cultures treated during 16h with 150µM H2O2 and 200nM C18:1-Leu. ROS, reactive oxygen species. n=10 per group, p=0.0048; one-sided Student's t-test. **b,** AlamarBlue cell viability assay of SH-SY5Y cells treated during 24h with 10µM thapsigargin and 200nM C18:1 -Leu. UPS, unfolded-protein stress. n=5 per group, p=0.0084; one-sided Student's t-test. Single values (experiments) are represented by dots. *p<0.05, and **p<0.01.

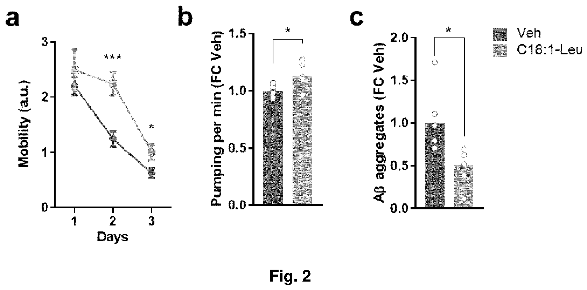

**Fig. 2****.** AD pathologies and phenotypes in GMC101 worms. **a,** Mobility assessment in vehicle and C18:1-Leu treated GMC101 worms during the 3 first days of adulthood. a.u., arbitrary units. n=75-100 worms per time, p=0.0001 and p=0.0152 for day 2 and 3, respectively; one-sided Student's t-test. **b,** Pumping per minute in vehicle and C18:1-Leu treated GMC101 worms at day 3. n=7 per group, p=0.0162; one-sided t-test. **c,** Relative amyloid aggregation in vehicle and C18:1-Leu treated GMC101 worms at day 1. n=6 experiments of -1000 worms each, pval=0.0108; one-sided Student's t-test. Single values (animals) are represented by dots. *p<0.05, and ***p<0.001.

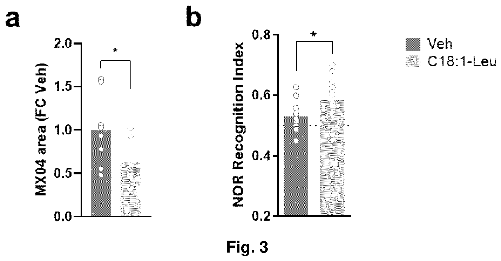

**Fig. 3****.** AD pathologies and phenotypes in APP/PS1 mice. a, Methoxy-X04 (MX04) IHC amyloid burden (area) quantification. n=8 per group, p=0.0283; one-sided Student's t-test. **b,** Novel object recognition (NOR) performance of vehicle and C18:1-Leu treated APP/PS1 mice. C18:1-Leu was provided from 15 to 18 months (m) of age. n=11-15 per group, p=0.0312. Single values (animals) are represented by dots. *p<0.05, **p<0.01, and ***p<0.001.

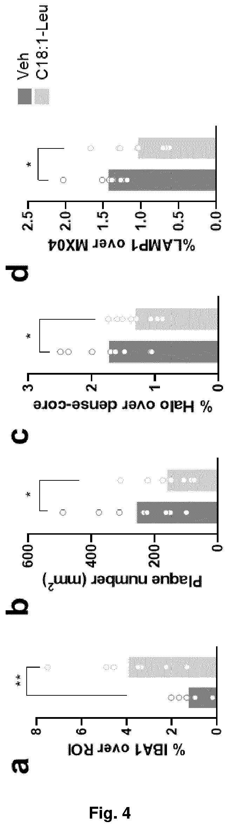

**Fig. 4****.** Microglia analysis in C18:1-Leu treated APP/PS1 mice. **a,** Quantification of Ionized calcium-binding adaptor molecule 1 (IBA1, microglia) area within methoxy-X04 (MX04, amyloid plaques) region of interest (ROI). n=6-7 per group, p=0.0051; one-sided Student's t-test. **b,** Amyloid plaque number quantification. n=8 per group, p=0.0444; one-sided Student's t-test. **c,** Percentage of amyloid halo (diffuse, toxic) area over plaque dense core. n=8 per group, p=0.0389; one-sided Student's t-test. **d,** Percentage of the *Lysosome-associated protein 1* (LAMP1, neuronal damage) area over to plaque dense core. n=8-9 per group, p=0.0132; one-sided Student's t-test. Single values (animals) are represented by dots. *p<0.05, and **p<0.01.

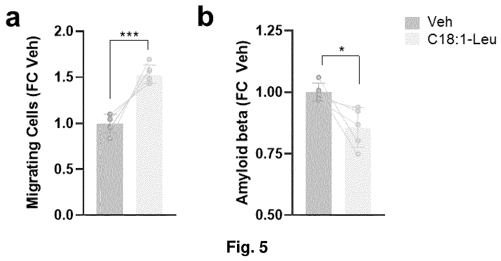

**Fig. 5****.** C18:1-Leu treatment in microglia cultures. **a,** Transwell assay of APP/PS1-derived primary microglia treated during 24h with 200nM C18:1-Leu. n=6 per group, p=0.0007; one-sided Student's t-test. **b,** Amyloid-beta 40 (Aβ40) ELISA assays of APP/PS1-derived primary microglia cultures after 24h of 200nM C18:1-Leu treatment in presence of amyloid-beta (Aβ). n=5 per group, p=0.0201; one-sided Student's t-test. Single values (experiments) are represented by dots. *p<0.05, **p<0.01, and ***p<0.001.

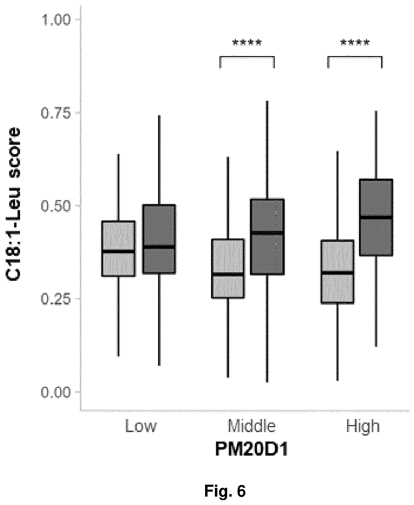

**Fig. 6****.** RNA expression signatures in human samples I. C18:1-Leu score according to PM20D1 haplotype in Alzheimer's disease (AD) and control postmortem brain samples. a, n=387 control and n=511 AD samples, Wilcoxon test, ****p<0.0001

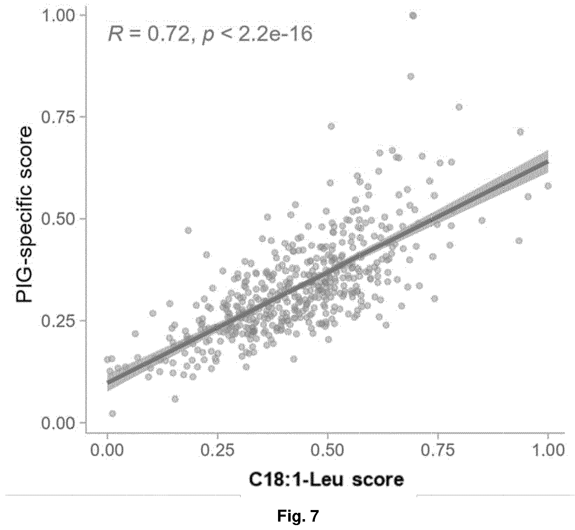

**Fig. 7****.** RNA expression signatures in human samples II. Correlation between PIG and C18:1-Leu scores in AD samples. n=511 AD samples, Spearman correlation.

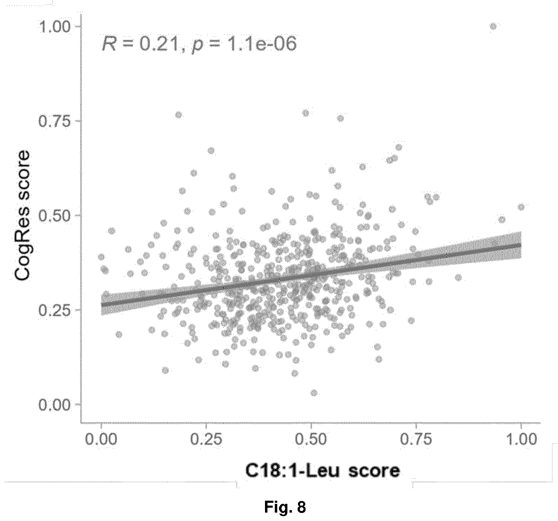

**Fig. 8****.** RNA expression signatures in human samples III. Correlation between RNA expression cognitive resilience (CogRes) and C18:1-Leu scores in AD samples. n=511 AD samples, Spearman correlation.

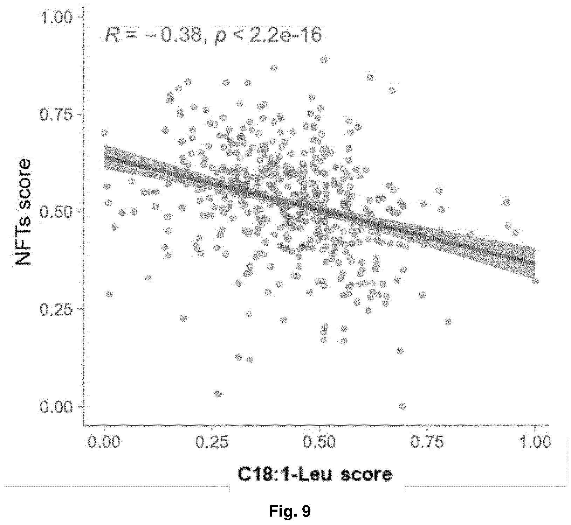

**Fig. 9****.** RNA expression signatures in human samples IV. Correlation between RNA expression cognitive resilience (CogRes) and C18:1-Leu scores in AD samples. n=511 AD samples, Spearman correlation.

### Examples

### Materials and Methods

### Mice

APP/PS1 male mice were standard housed on a 12h light-dark cycle with *ad libitum* access to food and water. C18:1-Leu (60 mg) and/or vehicle (1ml DMSO) was administered in alkaline drinking water (250ml) from 15 to 18 months of age. Mice were handled 5 min/day during five consecutive days the week before assaying cognition. The novel object recognition test was conducted in an open field arena of 42 × 30 cm. On the first day, mice were habituated for 10 min to the open arena. On the second day, mice were placed for 10 min in the arena with two identical objects, and the time exploring each object was automatically recorded (Ethovision, Noldus). On the third day, one of the two familiar objects was replaced by a novel object. The time exploring each of the objects was recorded and an object recognition index was calculated as the time spent exploring the novel object divided by the total time spent exploring the two objects (RI = timeₙₒᵥₑₗ / timeₙₒᵥₑₗ + time_{familiar} object). All animal procedures were conducted according to the Switzerland's and Spain's licenses VD2875 and 2020/VSC/PEA/0151, respectively.

### C. elegans

*C. elegans* were cultured at 20°C on nematode growth medium (NGM) agar plates seeded with E. coli strain OP50 unless stated otherwise. The strain used in this study was GMC101 (unc-54p::A-beta-1-42::unc-54 3'-UTR + mtl-2p::GFP), which was provided by the Caenorhabditis Genetics Center (University of Minnesota). The strain CL2122 was outcrossed 3 times in the N2 background, and subsequently used in the control experiments reported herein. C18:1-Leu was dissolved in DMSO and added just before pouring the plates. For phenotyping experiments, L4 worms were allowed to reach adulthood on the treatment plates. The worms were therefore exposed to the compound during adulthood until death, or up to 8 days. For protein analysis experiments, synchronized L1 worms were exposed to the compounds until harvest. Worms were harvested at day 1 to avoid any bias caused by worm's death. To ensure a permanent exposure to the compound, plates were changed twice a week. Mobility assay was performed as described (Mouchiroud, L. et al. The Movement Tracker: A Flexible System for Automated Movement Analysis in Invertebrate Model Organisms. Curr Protoc Neurosci 77, 8.37.1-8.37.21 (2016)), starting from day 1 of adulthood, using the Movement Tracker software. For paralysis and death score, 45 L4 worms per condition were allowed to reach adulthood on the treatment plates. Worms were manually scored for paralysis after poking, as already described (Mccoll, G. et al. Utility of an improved model of amyloid-beta (Aβ1-42) toxicity in Caenorhabditis elegans for drug screening for Alzheimer's disease. Mol Neurodegener 7, (2012)). Worms that were unable to respond to any, repeated stimulation, were scored as dead.

### Primary cultures

Primary hippocampal neuron cultures derived from post-natal day 0 (P0) APP/PS1 mice were cultured in media consisting of Neurobasal (Invitrogen), B27 supplement (Invitrogen), L-glutamine (Invitrogen) and penicillin/streptomycin (Invitrogen) (0.2 ml per well) on 96 wells plates (2.5×10⁴ cells per well) coated with Cultrex poly-L-lysine (Trevigen). On day *in vitro* 14 (DIV14) 200nM C18:1-Leu (Cayman) or vehicle was added, cells treated with 100µM H₂O₂ during 16h and cell viability measured by LDH release assay (Promega). For *in vitro* experiments, C18:1-Leu was used at 200nM according to previous publications (Long, J. Z. et al. The Secreted Enzyme PM20D1 Regulates Lipidated Amino Acid Uncouplers of Mitochondria. Cell 166, 424-435 (2016)). Primary astrocyte-microglia cultures derived from P3 APP/PS1 mice were cultured in MEM media (Gibco) supplemented with L-glutamine (Invitrogen), penicillin/streptomycin (Invitrogen) and 10% FBS (Cultek) on 75 cm² flasks (2×10⁶ cells). After two weeks of culture, microglia were harvested by gentle shaking. For transwell experiments, microglia were seed on 24 well transwell plates (6×10⁴ cells per well) in a total volume of 150 µl (upper chamber, insert 6,5mm transwell 8,0µm pet mem), with 800 µl of conditioned Aβ media obtained from primary cultures derived from APP/PS1 mice at DIV14 (lower chamber). C18:1-Leu or vehicle was added at the moment of seeding the cells. Next day, migrated cells were stained with DAPI, images acquired with the Motic AE31E microscope and quantified with Image J. For Aβ clearance experiments, microglia were seed on 96 well plates (2×10⁴ cells per well), coated with poly-D-lysine (Trevigen), and cultured with the media supplemented with GM-CSF (Sigma) during two days (0.2ml). On day three, equal amounts of conditioned Aβ media (10µl), plus C18:1-Leu (200nM) or vehicle, was added on the cells. On the fourth day, media was collected and remaining Aβ measured using the Amyloid beta 40 ELISA Kit (Novex).

### Cell lines

SH-SY5Y human neuroblastoma cells were cultured in media consisting of 4.5 g/L glucose Dulbecco Modified Eagle Medium (DMEM) (Biowest) supplemented with 15% FBS (Corning), 2 mM L-glutamine (Gibco) and 100 mg/ml penicillin/streptomycin (Gibco) (0.1 ml per well) on 96 wells plates (1×10⁴ cells per well). On the next day, 200nM C18:1-Leu or vehicle was added, cells treated with 10 µM Thapsigargin (Thermo Scientific) or 150 µM Hydrogen peroxide (Fisher) during 24h, and cell viability measured using AlamarBlue assay (Invitrogen).

### Synthesis of C18 :1-Leu

To a solution of Leucine (1 eq.) in acetone and water (1:1), K2CO3 (2 eq.) and oleoyl chloride (1.5 eq.) were added at 0° C. Then, the mixture was stirred at room temperature overnight. The reaction was then acidified with 1 M HCl until the pH4. The reaction mixture was extracted with ethyl acetate, and the organic layer was washed with brine (2X 200 mL) and dried over anhydrous Na2SO4. The solvent was dried under reduced pressure, and the crude product was purified on silica gel. The product was eluted at 30% of ethyl acetate in hexane, and oleoyl leucine was obtained as a sticky white solid (yield -60%). 1H NMR (400 MHz, CDCl3) δ 10.43 (s, broad, 1H), 6.19 (d, J = 8 Hz, 1H), 5.34-5.31 (m, 2H), 4.64-4.59 (m, 1H), 2.25 (t, J = 4 Hz, 2H), 2.01-1.97 (m, 4H), 1.72-1.57 (m, 5H), 1.28-1.25 (m, 20H), 0.95-0.85 (m, 9H). 13C NMR (CDCl3, 100 MHz) δ 176.51, 174.23, 50.88, 41.25, 36.47, 31.91, 29.77, 29.72, 29.53, 29.25, 29.19, 29.17, 27.23, 27.19, 25.68, 24.91, 22.86, 22.69, 21.89, 14.12. ESI-MS, calculated for C24H46NO3 [M+H]+: 396.34, found: 396.41. For the synthetic scheme, 1H and 13 NMR, and ESI-MS analysis see *Extended Data* *Fig. 1**.*

### Immunohistochemistry

Immunohistochemical analysis were performed on OCT embedded brains sections of 5-15 µm-thickness. Tissue sections were fixed with 4% paraformaldehyde (PFA) for 5 min, blocked with BSA 3% and 0,1% Triton X-100, and incubated with the following primary antibodies: CST3 (1:200, R&D systems), IBA1 (1:1000, Wako), and LAMP1 (1:100, BD biosciences), and the corresponding secondary antibodies. For IBA1 staining, primary antibody was incubated at room temperature in presence of 5% newborn calf serum. Amyloid plaques were stained with Methoxy-X04 (Abcam). Images were acquired with the confocal microscopes Olympus FV1000 IX81, Leica Spell, and Zeiss LSM 880-Airyscan, and analyzed with imaged Software.

### Western blot

For worms, a total of ≈1000 worms were collected and lysed by sonication with RIPA buffer containing protease and phosphatase inhibitors (Roche). For mice, entorhinal cortex samples were lysed by sonication with laemmli buffer (50mM Tris, 2% SDS, 2% Glycerol, 0.02% Bromophenol blue, pH 6.9). The concentration of extracted protein was determined by using the Bio-Rad DC Protein Assay and 5% Mercaptoethanol was added prior loading. Equal amounts of protein (15-30 µg/lane) were separated by SDS-PAGE (10-15%) and transferred onto nitrocellulose membranes (Amersham, GE Healthcare). Ponceau S staining (Sigma-Aldrich) was used for monitoring protein loading and transference. Nonspecific binding was blocked by incubation in 5% nonfat milk in phosphate-buffer saline containing 0.1% Tween (PBS) for 1 h at room temperature. Membranes were incubated overnight at 4 °C with the primary antibody rabbit anti-APP in PBS with 3% nonfat milk and 1 h in the corresponding horseradish-peroxidase-conjugated secondary antibody. Immunocomplexes were revealed by an enhanced chemiluminescence reagent (ECL Advance, Amersham Biosciences). Densitometric quantification was carried out by using imaged software. Each immunoblot experiment containing was repeated six times.

### RNA expression

RNA expression data from control and Alzheimer's disease postmortem brain samples were obtained from the NCBI Gene Expression Omnibus (GEO) database (GSE33000, GSE15222 and GSE48350) and analyzed using R software (http://www.R-project.org). Z-score normalization was applied prior to merging. Samples were stratified in three segments according to the corresponding high (methylated), mid (heterozygous methylated) and low (nonmethylated) expression PM20D1 haplotype - using the centroid-based clustering (K-means) method - as previously described (Sanchez-Mut, J. V et al. PM20D1 is a quantitative trait locus associated with Alzheimer's disease. Nat Med 24, 598-603 (2018)). N-oleoyl-L-Leucine (C18:1-Leu), Plaque-induced gene (PIG), cognitive reserve (CogRes) and neurofibrillary tangle (NFT) scores were calculated as the average of the scaled expression values (range 0 and 1) of the corresponding genes. C18:1-Leu genes were obtained through the comparison between single-cell RNA sequencing brain data from vehicle and C18:1-Leu treated mice (i.e., significantly upregulated genes, log-fold-change > 0.25 and adjusted pvalue < 0.05, in biologically relevant pathways, gProfiler adjusted p.value <0.05). PIG genes were obtained from Table S3 (Purple module) Chen et al (Chen, W. T. et al. Spatial Transcriptomics and In Situ Sequencing to Study Alzheimer's Disease. Cell 182, 976-991 e19 (2020)). CogRes genes were obtained from TableS2 Yegla and Foster⁷¹. NFT genes were obtained from TableS2 (upregulated genes in at least four of excitatory clusters 1, 2, 3, 7 and 10) Otero-Garcia et al. (Otero-Garcia, M. et al. Molecular signatures underlying neurofibrillary tangle susceptibility in Alzheimer's disease. Neuron 110, 2929-2948.e8 (2022)). Analysis code is available upon request.

### Statistics

Statistical analysis was done using R package and Prism 8.0 (GraphPad) as described in the figure legends. All experimental data points were included in the analysis, unless they were statistical outliers as determined by Grubb's outlier calculation's (GraphPad Prism). All *in vitro* and *in vivo* experiments were conducted by experimenters blinded to the experimental conditions and controlled for their normal distribution.

### Example 1

### PM20D1-derived N-oleoyl-L-Leucine (C18:1-Leu) reduces amyloid-beta production in primary neuronal cultures derived from AD mice

To begin to explore the potential role of C18:1-Leu in AD, it has been performed a series of in vitro assays. First, to investigate whether it modifies Aβ production, primary hippocampal neurons derived from AD mice were cultured in presence of vehicle or 200pm C18:1-Leu using ELISA assays. It has been observed that C18:1-Leu treatment reduces Aβ production in neuronal cells (Fig. 1a). Second, to determine whether it also protects against reactive oxygen species (ROS) and unfolded-protein stress (UPS), both of which are increased in AD (McLellan, M. E., Kajdasz, S. T., Hyman, B. T. & Bacskai, B. J. In vivo imaging of reactive oxygen species specifically associated with thioflavine S-positive amyloid plaques by multiphoton microscopy. J Neurosci 23, 2212-2217 (2003); Ajoolabady, A., Lindholm, D., Ren, J. & Pratico, D. ER stress and UPR in Alzheimer's disease: mechanisms, pathogenesis, treatments. Cell Death Dis 13, (2022)), were measured using hydrogen peroxide, thapsigargin and cell viability assays. It has been observed that C18:1-Leu treatment also increases neuroprotection against ROS (Fig. 1b) and UPS stress (Fig. 1c). Taken together, these results indicate that C18:1-Leu treatment reduces Aβ production and increases cell viability in cultured neuronal cells.

### Example 2

### PM20D1-derived N-oleoyl-L-Leucine (C18:1-Leu) improves AD phenotypes and pathologies in a C. elegans and mouse model of AD

To explore the protective role of PM20D1-derived N-oleoyl-L-Leucine (C18:1-Leu) *in vivo,* it has been taken advantage of two different AD models. The GMC101 strain of C. elegans, which aggregates Aβ peptides in the body wall of muscle cells after a temperature shift from 20 to 25ºC, impairing their mobility and viability (Mccoll, G. et al. Utility of an improved model of amyloid-beta (Aβ1-42) toxicity in Caenorhabditis elegans for drug screening for Alzheimer's disease. Mol Neurodegener 7, (2012)), and the APP/PS1 mouse, which shows a progressive accumulation of amyloid plaques, astrogliosis as well as learning and memory deficits.

It has been observed that 200 µM C18:1-Leu significantly improves AD-like phenotypes - i.e., spontaneous and induced mobility (Sorrentino, V. et al. Enhancing mitochondrial proteostasis reduces amyloid-β proteotoxicity. Nature 2017 552:7684 552, 187-193 (2017)) (Fig. 2a-c) - and amyloid pathologies (Fig. 2d) in GMC101 worms. Similarly, a 25 mg/kg/day per oral (p.o.) administration of C18:1-Leu during three months, starting at 15 months-of-age, improves amyloid burden and cognitive deficits in AD mice measured by immunohistochemistry (IHC) staining (Fig. 3a) and the novel object recognition test (NOR) (Fig. 3b). Taken together, these results indicate that C18:1-Leu treatment increases Aβ clearance and improves AD phenotypes in both C. elegans and mouse models of AD.

### Example 3

### C18:1-Leu treatment increases microglia association with amyloid plaques

Recent findings suggest a major role of microglia in AD (Sarlus, H. & Heneka, M. T. Microglia in Alzheimer's disease. J Clin Invest 127, 3240-3249 (2017)). To investigate the role of microglia in C18:1-Leu protective effects, it has been performed a series of IHC assays. It has been stained amyloid plaques with Methoxy-X04 (MX04) and microglia with Ionized calcium-binding adaptor molecule 1 (IBA1). It has been measured the relative MX04 area occupied by IBA1 and observed an increased microglia association in C18:1-Leu treated mice (Fig. 4a). Then, it has been sought to determine whether number and organization of amyloid plaques was also altered. Amyloid plaques are typically composed by a dense Aβ core surrounded by a halo of more soluble Aβ forms (Radde, R. et al. Aβ42-driven cerebral amyloidosis in transgenic mice reveals early and robust pathology. EMBO Rep 7, 940-946 (2006)). Microglia are known to be more effective at clearing the diffuse areas than the dense cores (Daria, A. et al. Young microglia restore amyloid plaque clearance of aged microglia. EMBO J 36, 583 (2017)). It has been observed that the number (Fig. 4b) and relative size of the diffuse halo of the plaques was reduced in C18:1-Leu treated mice (Fig. 4c). Next, since diffusible Aβ forms are also more reactive (Condello, C., Yuan, P., Schain, A. & Grutzendler, J. Microglia constitute a barrier that prevents neurotoxic protofibrillar Aβ42 hotspots around plaques. Nature Communications 2015 6:1 6, 1-14 (2015)), it has been examined neuronal damage by staining with Lysosome-associated protein 1 (LAMP1), which is increased in dystrophic neurites associated with amyloid plaques (Gowrishankar, S. et al. Massive accumulation of luminal protease-deficient axonal lysosomes at Alzheimer's disease amyloid plaques. Proc Natl Acad Sci U S A 112, E3699-E3708 (2015)), and observed a stronger LAMP1 area reduction in C18:1-Leu treated mice (Fig. 4d). Together, these data suggest that C18:1-Leu treatment effectively increases microglia association with amyloid plaques and reduces their size, number and toxicity.

### Example 4

### C18:1 -Leu treatment in vitro increases microglia Aβ chemotaxis and clearance, and neuronal viability against oxidative and unfolded protein stress

To support these observations on a functional level, it has been then performed a series of in vitro assays. First, it has been measured Aβ chemotaxis using a transwell migration assay (Chen, H. C. Boyden chamber assay. Methods Mol Biol 294, 15-22 (2005)). Briefly, APP/PS1 microglia were plated on a chamber sealed with a permeable membrane and their migration to a second chamber containing Aβ quantified. In line with the in vivo results, C18:1-Leu treatment increased Aβ chemotaxis in cultured microglia (Fig. 5a). Second, it has been investigated whether C18:1-Leu treatment also affected Aβ levels by ELISA, and observed that C18:1-Leu treatment increases Aβ clearance (Fig. 5b). These findings stipulate that C18:1-Leu treatment increases amyloid chemotaxis and clearance in microglia cells.

### Example 5

### C18:1-Leu RNA expression signatures are conserved in human postmortem samples

Finally, to determine whether similar effects can be observed in human patients, it has been performed a series of transcriptomic analysis using publicly available data from postmortem AD brains (GSE33000, GSE15222 and GSE48350). First, since PM20D1 is upregulated in AD (Sanchez-Mut, J. V, Glauser, L., Monk, D. & Graff, J. Comprehensive analysis of PM20D1 QTL in Alzheimer's disease. Clin Epigenetics 12, 20 (2020); Sanchez-Mut, J. V et al. PM20D1 is a quantitative trait locus associated with Alzheimer's disease. Nat Med 24, 598-603 (2018)), it has been tested if genes induced by C18:1-Leu in mice were also increased in AD samples. To do so, it has been performed a sequencing assay in C18:1-Leu treated mice and identified a series of differentially expressed genes (DEGs). Then, it has been tested whether these genes were also upregulated in human AD brains, particularly in PM20D1 high expression carriers (i.e., 48%, 71% and 76% for low, mid and high PM20D1 expression carriers, respectively) (Fig. 6). Second, to investigate their association with amyloid plaques, it has been assayed the expression of PIGs (Chen, W. T. et al. Spatial Transcriptomics and In Situ Sequencing to Study Alzheimer's Disease. Cell 182, 976-991 e19 (2020)) as proxy of microglia interaction with amyloid plaques. Both C18:1-Leu genes and PIG signatures were also highly correlated in human AD samples (Fig. 7). Finally, to investigate whether PM20D1/C18:1-Leu exert similar protective effects in human brains, it has been measured the expression of genes associated with cognitive reserve (CogRes) (Yegla, B. & Foster, T. C. Operationally defining cognitive reserve genes. Neurobiol Aging 110, 96-105 (2022)) and neurofibrillary tangle (NFT)-containing neurons (Otero-Garcia, M. et al. Molecular signatures underlying neurofibrillary tangle susceptibility in Alzheimer's disease. Neuron 110, 2929-2948.e8 (2022)) as proxies of cognition and neuronal damage, respectively. It has been found that CogRes genes positively correlated with the expression of PM20D1/C18:1-Leu induced genes, while NFT genes showed the opposite pattern (Fig. 8 and 9). Taken together, these results support the potential neuroprotective role of PM20D1/C18:1-Leu in human AD, and underscore their value to monitor and intercept the progression of the disease.

## Claims

1. N-oleoyl-L-Leucine (C18:1-Leu) for use in the treatment and/or prevention of a neurodegenerative disease.

2. The N-oleoyl-L-Leucine (C18:1-Leu) for the use according to claim 1, wherein the neurodegenerative disease is selected from Parkinson disease, multiple sclerosis disease, amyotrophic lateral sclerosis disease and Alzheimer's disease.

3. The N-oleoyl-L-Leucine (C18:1-Leu) for the use according to claim 2, wherein the neurodegenerative disease is Alzheimer's disease.
